# EUROPÄISCHE PATENTSCHRIFT

(11) **EP 2 032 519 B1**
(45) Veröffentlichungstag und Bekanntmachung des Hinweises auf die Patenterteilung: **31.07.2013**
(21) Anmeldenummer: 07726900.9
(22) Anmeldetag: 14.03.2007
(51) Int. Cl.: C07C 51/56, C07C 51/567, C07C 57/04

(54) **VERFAHREN ZUR KONTINUIERLICHEN HERSTELLUNG VON UNGESÄTTIGTEN CARBONSÄUREANHYDRIDEN**
METHOD FOR THE CONTINUOUS PRODUCTION OF UNSATURATED CARBOXYLIC ACID ANHYDRIDES
PROCÉDÉ DE PRODUCTION EN CONTINU D'ANHYDRIDES D'ACIDES CARBOXYLIQUES INSATURÉS

(30) Priorität: 23.06.2006 DE 102006029318
(43) Veröffentlichungstag der Anmeldung: 11.03.2009
(73) Patentinhaber: Evonik Röhm GmbH, 64293 Darmstadt (DE)
(72) Erfinder: BRÖLL, Dirk, 63225 Langen (DE); SIEGERT, Hermann, 64342 Seeheim-Jugenheim (DE)
(86) Internationale Anmeldenummer: PCT/EP2007/052409
(87) Internationale Veröffentlichungsnummer: WO 2007/147653

(56) Entgegenhaltungen:
- EP-A- 0 196 520

## Beschreibung

Die Erfindung beschreibt ein Verfahren zur kontinuierlichen Herstellung von ungesättigten Carbonsäureanhydriden, insbesondere die Reaktion einer ungesättigten Carbonsäure mit einem Keten.

In DE-A-3510035 wird ein Verfahren zur kontinuierlichen Herstellung von ungesättigten Carbonsäureanhydriden durch säurekatalysierte Umanhydridisierungsreaktion von Essigsäureanhydrid mit einer ungesättigten Carbonsäure im Mittelteil einer Destillationskolonne beschrieben. Zur Erreichung eines vollständigen Umsatzes wird Essigsäureanhydrid in einem Überschuss von 0.1 bis 0.5 Mol pro Mol Carbonsäure eingesetzt, wobei dann am Kolonnenkopf eine Mischung aus Essigsäure und Essigsäureanhydrid anfällt, also keine reine Essigsäure gewonnen wird.

Darüber hinaus wird das Produkt verunreinigt durch den Katalysator gebildet, der erst in einem weiteren Verfahrensschritt entfernt werden muss.

In US-A- 4,857,239 wird ein Verfahren zur Herstellung von Methacrylsäureanhydrid beschrieben, wobei das Molverhältnis von Methacrylsäure zu Essigsäureanhydrid 2.1 bis 3 beträgt und ein Polymerisationsinhibitor in die Destillationskolonne zugegeben wird. Gemäß den Beispielen ist das Verfahren diskontinuierlich. Nachteilig darüber hinaus ist, dass das im Überschuss eingesetzte Edukt ungenutzt anfällt.

In US-A-2003/0018217 wird ein diskontinuierliches Verfahren zur Herstellung von Methacrylsäureanhydrid beschrieben, wobei das anfängliche Molverhältnis von Methacrylsäure zu Essigsäureanhydrid bevorzugt 9 bis 11 beträgt. Die entstehende Essigsäure wird sofort abgeführt und der freiwerdende Reaktorinhalt mit Essigsäureanhydrid aufgefüllt. Zur Vermeidung einer Polymerisation werden in den Reaktor und in die Kolonne Inhibitoren zugegeben. Es werden viele Nebenprodukte gebildet, die sich nicht vollständig entfernen lassen. Aufgabe ist es nun ein verbessertes Verfahren zur kontinuierlichen Herstellung von ungesättigten Carbonsäureanhydriden bereitzustellen, bei dem ein vollständiger Umsatz der eingesetzten ungesättigten Carbonsäure erzielt wird und gleichzeitig das gebildete ungesättigte Carbonsäureanhydrid in hoher Reinheit gewonnen wird. Darüber hinaus soll die Polymerisation in allen Bereichen weitgehend vermieden werden und die Raum-Zeit-Ausbeute der Reaktion erhöht werden.
Gegenstand der Erfindung ist ein Verfahren zur kontinuierlichen Herstellung von ungesättigten Carbonsäureanhydriden der allgemeinen Formel I

R-C(O)-O-C(O)-R (I),

in der R einen ungesättigten organischen Rest mit 2 bis 12 C-Atomen darstellt,
durch Reaktion von einem Keten der allgemeinen Formel III

R'-CR"=C=O (III),

worin R' und R" gleich oder verschieden sind und Wasserstoff oder einen C₁ bis C₄-Alkylrest darstellen,
mit einer ungesättigten Carbonsäure der allgemeinen Formel II

R-COOH (II),

in der R die oben angegebene Bedeutung hat,
dadurch gekennzeichnet, dass in einer Apparatur, enthaltend einen Reaktionsbereich (1) für die Reaktion von einem Keten mit einer ungesättigten Carbonsäure der allgemeinen Formel II,
einen Reaktionsbereich (2) für die Weiterreaktion der gebildeten Rohanhydridmischung und
eine Rektifikationskolonne mit einem oberen, mittleren und unteren Bereich, in deren Sumpf ein inertes Siedeöl, eine hochsiedende, inerte Substanz mit einem Siedepunkt höher als die Siedepunkte der an der Reaktion beteiligten Komponenten, vorgelegt ist,
a) die ungesättigte Carbonsäure der allgemeinen Formel II in den Reaktionsbereich (1) eingespeist wird,
b) die entstandene Rohanhydridmischung aus Schritt a) in einen weiteren
   außerhalb und/oder innerhalb der Kolonne befindlichen und/oder im Reaktionsbereich (1) enthaltenen Reaktionsbereich (2) eingespeist wird,
c) die Rohanhydridmischung aus Schritt b) in der Rektifikationskolonne aufgereinigt wird,
d) am Kopf der Kolonne die entstehende Carbonsäure abgezogen und zur Ketengewinnung wieder zurückgeführt wird,
e) die nicht umgesetzten Edukte und gebildete Zwischenprodukte in den Reaktionsbereich (1) und/oder (2) zurückgeführt werden und
f) das Produkt der Formel I zwischen dem mittleren und unteren Bereich der Rektifikationskolonne erhalten wird.

Durch diese technischen Merkmale wird erreicht, dass ein vollständiger Umsatz der Edukte und gleichzeitig eine hohe Reinheit der Produkte erzielt wird und die Polymerisation in allen Bereichen weitgehend vermieden wird, da u.a. lange Verweilzeiten des gebildeten ungesättigten Anhydrids im Kolonnensumpf ausgeschlossen werden.

Für das erfindungsgemäße Verfahren geeignete ungesättigte Carbonsäuren weisen einen ungesättigten organischen Rest mit 2 bis 12, vorzugsweise 2 bis 6, besonders bevorzugt 2 bis 4 Kohlenstoffatomen auf. Geeignete Alkenylgruppen sind die Vinyl-, Allyl-, 2-Methyl-2-propen-, 2-Butenyl-, 2-Pentenyl-, 2-Decenyl, 1-Undecenyl und die 9,12-Octadecadienyl-Gruppe. Besonders bevorzugt sind die Vinyl- und die Allylgruppe.

Zu den besonders bevorzugten Carbonsäuren gehören u.a. (Meth)acrylsäuren. Der Begriff (Meth)acrylsäuren ist in der Fachwelt bekannt, wobei hierunter neben Acrylsäure und Methacrylsäure auch Derivate dieser Säuren zu verstehen sind. Zu diesen Derivaten gehören unter anderem β-Methylacrylsäure (Butensäure, Crotonsäure), α, β-Dimethylacrylsäure, β-Ethylacrylsäure, α-Chloracrylsäure, α-Cyanacrylsäure, 1-(Trifluormethyl)acrylsäure, sowie β,β-Dimethylacrylsäure. Bevorzugt sind Acrylsäure (Propensäure) und Methacrylsäure (2-Methylpropensäure).

Geeignete Ketene für das erfindungsgemäße Verfahren besitzen die allgemeine Formel III R'-CR"=C=O (III), worin R' und R" gleich oder verschieden sind und Wasserstoff oder einen C₁ bis C₄-Alkylrest darstellen. Vorzugsweise wird als Keten CH₂=C=O verwendet.

Die Herstellung von Keten erfolgt nach gängigen Verfahren, die aus der allgemeinen Fachliteratur, beispielsweise aus H. Held, A. Rengstl und D. Mayer, Acetic Anhydride and Mixed Fatty Acid Anhydrides in Ullmann's Encyclopedia of Industrial Chemistry, 6. Aufl., Wiley VCH, Weinheim, 2003, S.184-185, bekannt sind.

Bei dem erfindungsgemäßen Verfahren wird das als Reaktant verwendete Keten durch thermische Spaltung von einer Carbonsäure der allgemeinen Formel IV R'-CR"H-COOH (IV), worin R' und R" die obengenannte Bedeutung haben, in einem Ketenofen in Anwesenheit eines üblichen Katalysators wie beispielsweise Triethylphosphat erhalten. Die thermische Spaltung erfolgt unter allgemein üblichen Temperatur- und Druckverhältnissen.

Bevorzugt ist die thermische Spaltung von Essigsäure, wobei als Keten CH₂=C=O erhalten wird.

Die für das erfindungsgemäße Verfahren geeignete Apparatur weist einen außerhalb der Rektifikationskolonne befindlichen Reaktionsbereich oder Reaktor (1) auf, wobei dieser Bereich mit den weiteren Bestandteilen der Apparatur verbunden ist.

Das gewonnene Keten wird nach üblichen Methoden abgetrennt und mit einer ungesättigten Carbonsäure der allgemeinen Formel II R-COOH (II), worin R die obengenannte Bedeutung hat, in dem Reaktionsbereich (1) umgesetzt. Die erhaltene Rohanhydridmischung reagiert in einem Reaktionsbereich oder Reaktor (2) weiter. Der Reaktionsbereich (2) kann außerhalb und/oder innerhalb der Rektifikationskolonne und/oder im Reaktionsbereich (1) mit enthalten sein.

Vorzugsweise befindet sich der Reaktionsbereich (2) innerhalb oder außerhalb der Rektifikationskolonne, besonders bevorzugt außerhalb. In diesem Reaktionsbereich (2) ist vorzugsweise mindestens ein Katalysator vorgesehen.

Das molare Verhältnis der Reaktanten, das heißt von ungesättigter Carbonsäure der Formel II zu Keten der Formel III beträgt üblicherweise 1:4 bis 8:1, vorzugsweise 2:1.

Die Reaktion wird im Reaktionsbereich (2) bevorzugt bei Temperaturen im Bereich von 30 bis 120 °C, besonders bevorzugt bei 40 bis 100 °C, insbesondere bei 50 bis 80 °C, durchgeführt. Dabei ist die Reaktionstemperatur abhängig vom eingestellten Systemdruck. Falls Reaktionsbereich (2) innerhalb der Kolonne angeordnet ist, wird die Reaktion vorzugsweise im Druckbereich von 5 bis 100 mbar (absolut), insbesondere bei 10 bis 50 mbar (absolut) und besonders bevorzugt bei 20 bis 40 mbar (absolut) durchgeführt.

Falls sich Reaktionsbereich (2) außerhalb der Kolonne und separat von Reaktionsbereich (1) befindet, können dort andere Druck- und Temperaturverhältnisse gewählt werden. Das hat den Vorteil, dass die Reaktionsparameter des Reaktors (2) unabhängig von den Betriebsbedingungen in der Kolonne und im Reaktionsbereich (1) eingestellt werden können.

Die Reaktionsdauer hängt von der Reaktionstemperatur ab; die Verweilzeit im Reaktionsbereich (2) bei einmaligem Durchgang beträgt vorzugsweise 0,5 bis 15 Minuten und besonders bevorzugt 1 bis 5 Minuten.

Bei der Herstellung von (Meth)acrylsäureanhydrid aus Keten CH₂=C=O und (Meth)acrylsäure beträgt die Reaktionstemperatur im Reaktionsbereich (2) vorzugsweise 40 bis 100 °C, besonders bevorzugt 50 bis 90 °C und ganz besonders bevorzugt 70 bis 85 °C.

Die Reaktionsmischung kann neben den Reaktanten weitere Bestandteile, wie beispielsweise Lösungsmittel, Katalysatoren und Polymerisationsinhibitoren umfassen.

Falls ein Katalysator innerhalb des Reaktionsbereiches (2), innerhalb der Rektifikationskolonne eingesetzt wird, kann dieser Katalysator in jedem Bereich der Rektifikationskolonne vorgesehen sein, bevorzugt im mittleren Bereich.

Vorzugsweise wird der Katalysator jedoch in einem außerhalb der Kolonne befindlichen und separat von Reaktionsbereich (1) angeordneten Reaktionsbereich (2) bereitgestellt. Diese Anordnung des Katalysatorbereichs ist bevorzugt, wobei die Rohanhydridmischung ständig durch den Katalysatorbereich geleitet wird. Hierdurch entsteht kontinuierlich das ungesättigte Carbonsäureanhydrid der Formel I, beispielsweise (Meth)acrylsäureanhydrid, sowie eine Carbonsäure der Formel IV, beispielsweise Essigsäure, die zur Ketengewinnung zurückgeführt wird.

Bevorzugt werden in dem Reaktionsbereich (2) heterogene Katalysatoren verwendet. Als heterogene Katalysatoren besonders geeignet sind saure Festbettkatalysatoren, insbesondere saure Ionenaustauscher (Kationenaustauscher).

Zu den besonders geeigneten sauren Ionenaustauschern gehören insbesondere Kationenaustauscherharze wie sulfonsäuregruppenhaltige Styrol-Divinylbenzolpolymere. Geeignete Kationenaustauscherharze können kommerziell von Rohm&Haas unter der Handelsbezeichnung Amberlyst^{®}, von Dow unter der Handelsbezeichung Dowex^{®} und von Lanxess unter der Handelsbezeichnung Lewatit^{®} erhalten werden.

Die Katalysatormenge in L beträgt vorzugsweise 1/10 bis das 2fache, besonders bevorzugt 1/5 bis 1/2, der zu produzierenden Menge an ungesättigten Carbonsäureanhydrid der Formel I in L/h.

Zu den bevorzugt einsetzbaren Polymerisationsinhibitoren gehören unter anderem Octadecyl-3-(3,5-di-tert-butyl-4-hydroxyphenyl)-propionat, Phenothiazin, Hydrochinon, Hydrochinonmonomethylether, 4-Hydroxy-2,2,6,6-tetramethylpiperidinooxyl (TEMPOL), 2,4-Dimethyl-6-tert-butylphenol, 2,6-Di-tert-butylphenol, 2,6-Di-tert-butyl-4-methylphenol, para-substituierte Phenylendiamine wie z. B. N,N'-Diphenyl-p-phenylendiamin, 1,4-Benzochinon, 2,6-Di-tert-butyl-alpha-(dimethylamino)-p-cresol, 2,5-Di-tert-butylhydrochinon oder Gemische aus zwei oder mehreren dieser Stabilisatoren. Ganz besonders bevorzugt ist Phenothiazin.

Die Zudosierung des Inhibitors kann in den Feed der ungesättigten Carbonsäure der allgemeinen Formel II, vor den Reaktionsbereich (1), vor den Reaktionsbereich (2) und/oder in die Rektifikationskolonne, vorzugsweise an deren Kopf, erfolgen.

In der Rektifikationskolonne wird das ungesättigte Carbonsäureanhydrid als Zielprodukt zwischen dem mittleren und unteren Bereich bevorzugt gasförmig abgezogen. Die Rückführung nicht umgesetzter Edukte und gebildeter Zwischenprodukte in den Reaktionsbereich erfolgt zum Beispiel mittels einer Pumpe.

Aus dem Kolonnensumpf können Hochsieder wie zugesetzte Inhibitoren durch übliche Methoden ausgeschleust werden, beispielsweise durch einen Dünnschichtverdampfer oder einen ähnliche Aufgaben verrichtenden Apparat, der verdampfende Stoffe in die Rektifikationskolonne zurückführt und nicht verdampfende Hochsieder ausschleust.

Für das erfindungsgemäße Verfahren wird als Siedeöl eine hochsiedende, inerte thermisch langzeitstabile Substanz mit einem Siedepunkt höher als die Siedepunkte der an der Reaktion beteiligten Komponenten verwendet, um die destillative Abtrennung des gebildeten Säureanhydrids ohne Polymerisation zu gewährleisten. Der Siedepunkt des Siedöls sollte aber auch nicht zu hoch sein, um die thermische Belastung des gebildeten Säureanhydrids zu reduzieren. Das Siedeöl befindet sich im Sumpf der Kolonne, um lange Verweilzeiten des polymerisationsanfälligen Zielproduktes zu vermeiden.

Im Allgemeinen liegt die Siedetemperatur des Siedeöls bei Normaldruck (1013 mbar) bei 200 bis 400 °C, insbesondere bei 240 bis 290 °C.

Geeignete Siedeöle sind u.a. höherkettige unverzweigte Paraffine mit 12 bis 20 Kohlenstoffatomen, aromatische Verbindungen wie Diphyl (eutektische Mischung aus 75 % Biphenyloxid und 25 % Biphenyl), alkylsubstituierte Phenole oder Naphthalinverbindungen, Sulfolan (Tetrahydro-thiophen-1,1-dioxid) oder Mischungen aus diesen.

Geeignete Beispiele sind die nachfolgend aufgeführten Siedeöle:

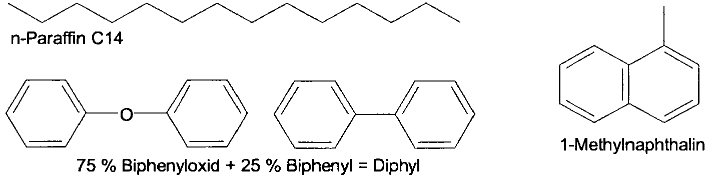

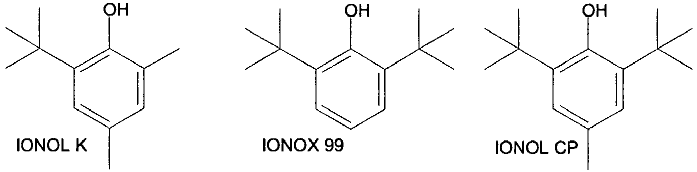

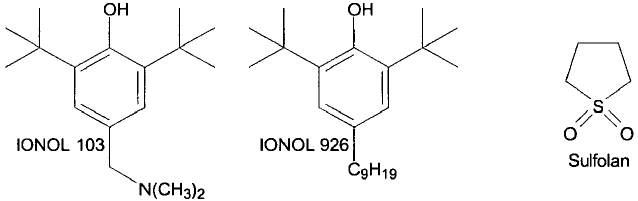

Besonders bevorzugt werden 2,6-di-tert-Butyl-para-cresol, 2,6-di-tert-Butyl-phenol, Sulfolan, Diphyl oder Mischungen aus diesen eingesetzt, ganz besonders bevorzugt Sulfolan.

Für die Aufreinigung der Rohanhydridmischung gemäß der vorliegenden Erfindung kann jede Rektifikationskolonne verwendet werden, die im oberen, mittleren und unteren Bereich je 5 bis 15 Trennstufen besitzt. Bevorzugt beträgt die Zahl der Trennstufen im oberen Bereich 10 bis 15 und im mittleren und unteren Bereich 8 bis 13. Als Anzahl der Trennstufen wird in der vorliegenden Erfindung die Anzahl der Böden bei einer Bodenkolonne multipliziert mit dem Bodenwirkungsgrad oder die Anzahl der theoretischen Trennstufen im Fall einer Packungskolonne oder eine Kolonne mit Füllkörpern bezeichnet.

Beispiele für eine Rektifikationskolonne mit Böden beinhalten solche wie Glockenböden, Siebböden, Tunnelböden, Ventilböden, Schlitzböden, Sieb-Schlitzböden, Sieb-Glockenböden, Düsenböden, Zentrifugalböden, für eine Rektifikationskolonne mit Füllkörper solche wie Raschig-Ringe, Lessing-Ringe, Pall-Ringe, Berl-Sättel, Intalox Sättel und für eine Rektifikationskolonne mit Packungen solche wie vom Typ Mellapak (Sulzer), Rombopak (Kühni), Montz-Pak (Montz) und Packungen mit Katalysatortaschen, beispielsweise Katapak (Sulzer).

Eine Rektifikationskolonne mit Kombinationen aus Bereichen von Böden, aus Bereichen von Füllkörpern und/oder aus Bereichen von Packungen kann ebenso verwendet werden.

Bevorzugt wird eine Rektifikationskolonne mit Füllkörpern und/oder Packungen eingesetzt.

Die Rektifikationskolonne kann aus jedem hierfür geeigneten Material hergestellt werden. Hierzu gehören u.a. Edelstahl sowie inerte Materialien.

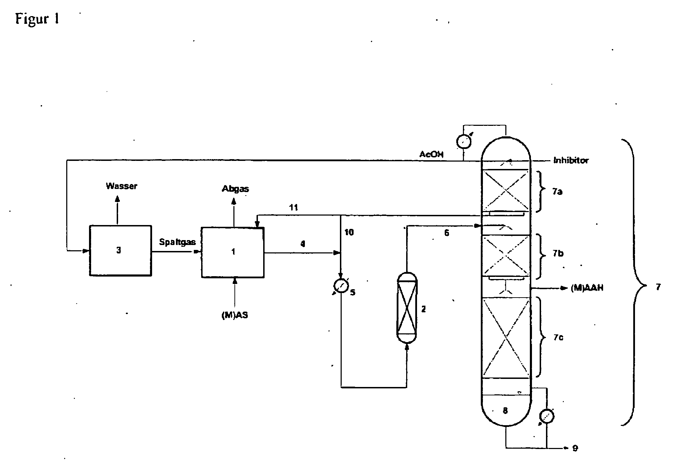

Eine bevorzugte Ausführungsform des erfindungsgemäßen Verfahrens ist in Fig. 1 schematisch dargelegt.

Die Herstellung von Keten (3) erfolgt nach einem gängigen Verfahren. Eine detaillierte Beschreibung der Ketenherstellung beinhaltet z.B. H. Held, A. Rengstl und D. Mayer, Acetic Anhydride and Mixed Fatty Acid Anhydrides in Ullmann's Encyclopedia of Industrial Chemistry, 6. Aufl., Wiley VCH, Weinheim, 2003, S. 184-185.

Die nachfolgende Umsetzung von Keten mit (Meth)acrylsäure (= (M)AS) erfolgt in einem Reaktionsbereich (1) ebenfalls nach einem gängigen Verfahren (s.o. H. Held et al., S. 185 ff.). Die Rohanhydridmischung (4) wird nun einem weiteren Reaktionsbereich (2) zugeführt. Die Temperatur der Reaktanten kann dabei über einen Wärmetauscher (5) in der Zuführung eingestellt werden.

Der Reaktor (2) ist vorzugsweise ein Strömungsrohrreaktor, der einen Festbettkatalysator enthält. Als Festbettkatalysator wird bevorzugt ein saurer Ionenaustauscher eingesetzt.

Der Reaktorabstrom (6) wird in die Rektifikationskolonne (7) eingespeist, vorzugsweise unterhalb des Rücklaufstromes aus dem oberen Bereich (7a) der Kolonne. In der Kolonne (7) findet die Trennung der Komponenten statt. Zur Vermeidung von Polymerisation wird vorzugsweise am Kopf der Kolonne Inhibitor und in den (M)AS-Feed zudosiert.

Im oberen Bereich (7a) wird die leichtsiedende Essigsäure von den Mittelsiedern ((M)AS, Zwischenprodukte) abgetrennt, am Kopf abgezogen und zur Ketenherstellung wieder zurückgeführt. Im mittleren Bereich (7b) der Kolonne findet die Trennung Mittelsieder gegen (Meth)acrylsäureanhydrid (= (M)AAH) statt, wobei zwischen dem mittleren und unteren Teil (M)AAH bevorzugt gasförmig abgezogen wird. Im unteren Bereich (7c) der Kolonne wird (M)AAH von dem im Sumpf befindlichen Siedeöl (8) getrennt. Im Sumpf befindliche Hochsieder können durch übliche Methoden ausgeschleust werden (9), beispielsweise durch einen Dünnschichtverdampfer oder einen ähnliche Aufgaben verrichtenden Apparat, der verdampfende Stoffe in die Rektifikationskolonne zurückführt und nicht verdampfende Hochsieder ausschleust.

Der aus dem oberen Bereich (7a) resultierende Flüssigkeitsstrom wird vollständig aus der Kolonne abgezogen und als Kreislaufstrom (10) zusammen mit dem Rohanhydridstrom (4) dem Reaktor (2) zugeführt. Alternativ kann der Kreislaufstrom (10) vollständig oder teilweise auch dem Reaktionsbereich (1) über Leitung (11) zugeführt werden.

## Patentansprüche

1. Verfahren zur kontinuierlichen Herstellung von ungesättigten Carbonsäureanhydriden der allgemeinen Formel I
R-C(O)-O-C(O)-R (I),
in der R einen ungesättigten organischen Rest mit 2 bis 12 C-Atomen darstellt, durch Reaktion von einem Keten der allgemeinen Formel III
R'-CR"=C=O (III)
worin R' und R" gleich oder verschieden sind und Wasserstoff oder einen C₁ bis C₄-Alkylrest darstellen,
mit einer ungesättigten Carbonsäure der allgemeinen Formel II
R-COOH (II),
in der R die oben angegebene Bedeutung hat,
**dadurch gekennzeichnet, dass** in einer Apparatur, enthaltend
einen Reaktionsbereich (1) für die Reaktion von einem Keten der allgemeinen Formel III mit einer ungesättigten Carbonsäure der allgemeinen Formel II,
einen Reaktionsbereich (2) für die Weiterreaktion der gebildeten Rohanhydridmischung und
eine Rektifikationskolonne mit einem oberen, mittleren und unteren Bereich, in deren Sumpf ein inertes Siedeöl, eine hochsiedende, inerte Substanz mit einem Siedepunkt höher als die Siedepunkte der an der Reaktion beteiligten Komponenten, vorgelegt ist
a) die ungesättigte Carbonsäure der allgemeinen Formel II in den Reaktionsbereich (1) eingespeist wird,
b) die entstandene Rohanhydridmischung aus Schritt a) in einen weiteren außerhalb und/oder innerhalb der Kolonne befindlichen und/oder im Reaktionsbereich (1) enthaltenen Reaktionsbereich (2) eingespeist wird,
c) die Rohanhydridmischung aus Schritt b) in der Rektifikationskolonne aufgereinigt wird,
d) am Kopf der Kolonne die entstehende Carbonsäure abgezogen und zur Ketengewinnung wieder zurückgeführt wird,
e) die nicht umgesetzten Edukte und gebildete Zwischenprodukte in den Reaktionsbereich (1) und/oder (2) zurückgeführt werden und
f) das Produkt der Formel I zwischen dem mittleren und unteren Bereich der Rektifikationskolonne erhalten wird.

2. Verfahren gemäß Anspruch 1, **dadurch gekennzeichnet, dass** in dem Reaktionsbereich (2) ein heterogener Katalysator verwendet wird.

3. Verfahren gemäß Anspruch 2, **dadurch gekennzeichnet, dass** ein saurer Festbettkatalysator verwendet wird.

4. Verfahren gemäß Anspruch 2 oder 3, **dadurch gekennzeichnet, dass** ein Kationenaustauscher als Katalysator verwendet wird.

5. Verfahren gemäß einem der Ansprüche 1 bis 4, **dadurch gekennzeichnet, dass** sich der Reaktionsbereich (2) nur außerhalb der Kolonne und separat von Reaktionsbereich (1) befindet.

6. Verfahren gemäß einem der Ansprüche 1 bis 5, **dadurch gekennzeichnet, dass** das ungesättigte Carbonsäureanhydrid der Formel I (Meth)acrylsäureanhydrid ist, hergestellt durch Reaktion von einem Keten der Formel CH₂=C=O und (Meth)acrylsäure.

7. Verfahren gemäß einem der Ansprüche 1 bis 6, **dadurch gekennzeichnet, dass** als Siedeöl 2,6-di-tert-Butyl-para-cresol, 2,6-di-tert-Butyl-phenol, Sulfolan oder Diphyl oder Mischungen aus diesen verwendet werden.

8. Verfahren gemäß einem der Ansprüche 1 bis 7, **dadurch gekennzeichnet, dass** als Siedeöl Sulfolan verwendet wird.

9. Verfahren gemäß einem der Ansprüche 1 bis 8, **dadurch gekennzeichnet, dass** aus dem Kolonnensumpf hochsiedende Komponenten ausgeschleust werden und verdampfende Stoffe in die Kolonne zurückgeführt werden.

## Claims

1. Process for the continuous preparation of unsaturated carboxylic anhydrides of the general formula I
R-C(O)-O-C(O)-R (I),
where R is an unsaturated organic radical having from 2 to 12 carbon atoms,
by reaction of a ketene of the general formula III
R'-CR"=C=O (III),
where R' and R" are identical or different and are each hydrogen or a C₁-C₄-alkyl radical with an unsaturated carboxylic acid of the general formula II
R-COOH (II),
where R is as defined above,
**characterized in that**, in an apparatus containing
a reaction region (1) for the reaction of a ketene of the general formula III with an unsaturated carboxylic acid of the general formula II,
a reaction region (2) for the further reaction of the crude anhydride mixture formed and
a rectification column which has an upper, middle and lower region and whose bottom has been charged with an inert vaporization oil, a high-boiling, inert substance having a boiling point higher than the boiling points of the components participating in the reaction,
a) the unsaturated carboxylic acid of the general formula II is fed into the reaction region (1),
b) the crude anhydride mixture formed in step a) is fed into a further reaction region (2) which is located outside and/or within the column and/or is present in the reaction region (1),
c) the crude anhydride mixture from step b) is purified in the rectification column,
d) the carboxylic acid formed is taken off at the top of the column and is recirculated to ketene production,
e) the unreacted starting materials and intermediates formed are recirculated to the reaction region (1) and/or (2) and
f) the product of the formula I is obtained between the middle and lower regions of the rectification column.

2. Process according to Claim 1, **characterized in that** a heterogeneous catalyst is used in the reaction region (2).

3. Process according to Claim 2, **characterized in that** an acidic fixed-bed catalyst is used.

4. Process according to Claim 2 or 3, **characterized in that** a cation exchanger is used as catalyst.

5. Process according to any of Claims 1 to 4, **characterized in that** the reaction region (2) is located only outside the column and is separate from the reaction region (1).

6. Process according to any of Claims 1 to 5, **characterized in that** the unsaturated carboxylic anhydride of the formula I is (meth)acrylic anhydride prepared by reaction of a ketene of the formula CH₂=C=O and (meth)acrylic acid.

7. Process according to any of Claims 1 to 6, **characterized in that** 2,6-di-tert-butyl-para-cresol, 2,6-di-tert-butylphenol, sulfolane or Diphyl or a mixture of these is used as vaporization oil.

8. Process according to any of Claims 1 to 7, **characterized in that** sulfolane is used as vaporization oil.

9. Process according to any of Claims 1 to 8, **characterized in that** high-boiling components are removed from the bottoms from the column and vaporizing substances are recirculated to the column.

## Revendications

1. Procédé pour la production en continu d'anhydrides d'acides carboxyliques insaturés de formule générale I
R-C(O)-O-C(O)-R (I),
dans laquelle R représente un radical organique insaturé ayant de 2 à 12 atomes de carbone,
par réaction d'un cétène de formule générale II
R'-CR"=C=O (III)
dans laquelle R' et R" sont identiques ou différents et représentent un atome d'hydrogène ou un radical alkyle en C₁-C₄,
avec un acide carboxylique insaturé de formule générale II
R-COOH (II)
dans laquelle R a la signification indiquée plus haut,
**caractérisé en ce que** dans un appareil, comportant une zone de réaction (1) pour la réaction d'un cétène de formule générale III avec un acide carboxylique insaturé de formule générale II,
une zone de réaction (2) pour la réaction subséquente du mélange contenant l'anhydride brut formé et
une colonne de rectification comportant une zone supérieure, une zone médiane et une zone inférieure, dans le fond de laquelle est disposée au préalable une huile bouillante inerte, une substance inerte à haut point d'ébullition, ayant un point d'ébullition supérieur aux points d'ébullition des composants participant à la réaction,
a) l'acide carboxylique insaturé de formule générale II est introduit dans la zone de réaction (1),
b) le mélange contenant l'anhydride brut résultant, provenant de l'étape a), est introduit dans une autre zone de réaction (2) se trouvant à l'extérieur et/ou à l'intérieur de la colonne et/ou contenue dans la zone de réaction (1),
c) le mélange contenant l'anhydride brut, provenant de l'étape b), est purifié dans la colonne de rectification,
d) l'acide carboxylique produit à la tête de la colonne est évacué et renvoyé de nouveau à la préparation de cétène,
e) les produits de départ n'ayant pas réagi et les produits intermédiaires formés sont renvoyés dans la zone de réaction (1) et/ou la zone de réaction (2) et
f) on obtient le produit de formule I entre la zone médiane et la zone inférieure de la colonne de rectification.

2. Procédé selon la revendication 1, **caractérisé en ce que** dans la zone de réaction (2) on utilise un catalyseur hétérogène.

3. Procédé selon la revendication 2, **caractérisé en ce qu'**on utilise un catalyseur acide en lit fixe.

4. Procédé selon la revendication 2 ou 3, **caractérisé en ce qu'**on utilise comme catalyseur un échangeur de cations.

5. Procédé selon l'une quelconque des revendications 1 à 4, **caractérisé en ce que** la zone de réaction (2) se trouve uniquement à l'extérieur de la colonne et séparément de la zone de réaction (1).

6. Procédé selon l'une quelconque des revendications 1 à 5, **caractérisé en ce que** l'anhydride d'acide carboxylique insaturé de formule I est l'anhydride (méth)acrylique, produit par réaction d'un cétène de formule CH₂=C=O et d'acide (méth)acrylique.

7. Procédé selon l'une quelconque des revendications 1 à 6, **caractérisé en ce qu'**on utilise comme huile bouillante le 2,6-di-tert-butyl-para-crésol, le 2,6-di-tert-butyl-phénol, le sulfolane ou le Diphyl ou des mélanges de ceux-ci.

8. Procédé selon l'une quelconque des revendications 1 à 7, **caractérisé en ce qu'**on utilise comme huile bouillante le sulfolane.

9. Procédé selon l'une quelconque des revendications 1 à 8, **caractérisé en ce que** les composants à haut point d'ébullition sont évacués du pied de la colonne et les substances vaporisées sont renvoyées dans la colonne.
